# EUROPEAN PATENT APPLICATION

(11) **EP 3 284 484 A1**
(43) Date of publication of application: **21.02.2018**
(21) Application number: 16780326.1
(22) Date of filing: 15.04.2016
(51) Int. Cl.: A61K 47/38, A61K 9/06, A61K 9/08

(54) **TRANSDERMAL PREPARATION CONTAINING ANTIFUNGAL ACTIVE MATERIAL**

(30) Priority: 17.04.2015 KR 20150054524
(71) Applicant: Dong-A Pharmaceutical Co., Ltd., Seoul 02587 (KR)
(72) Inventor: SOHN, Mi Won, Yongin-si Gyeonggi-do 16822 (KR); JUN, Joon Ho, Yongin-si Gyeonggi-do 17073 (KR); HAN, Sang Dug, Yongin-si Gyeonggi-do 16951 (KR); YU, Jae Young, Yongin-si Gyeonggi-do 17081 (KR); KU, Wan Sung, Anyang-si Gyeonggi-do 14075 (KR); CHOI, Sung Rak, Incheon 22224 (KR)
(74) Representative: Lavoix
(86) International application number: PCT/KR2016/003952
(87) International publication number: WO 2016/167603

(57) **Abstract**

The antifungal transdermal pharmaceutical composition of present invention containing an antifungal agent, a hydrophilic agent and a hydrophilic solvent and the method of preparing the same are provided. The composition has an excellent effect on hyper-keratinized skin such as intractable psoriasis and/or chronic keratinized athlete's foot which are not be permeated deeply by drug, by performing the deep penetration of antifungal agent into skin at a high concentration. In addition, the composition can be absorbed at a high concentration in skin, thereby reducing the administration frequency. The composition can deliver drug in a short time by forming hydrophilic film at the lesion region, and can reduce the inconvenience of use, in which the produced film is not washed well, because it is washed well in water.

## Description

### [Technical Field]

The present invention relates to a transdermal formulation comprising an antifungal agent and a preparation method thereof, and more specifically, to an antifungal transdermal formulation for increasing an skin permeation amount of an antifungal agent, reducing an administration frequency, and being easily washed with water.

### [Related Art]

Dermatophytosis or tinea is a collective term for skin surface fungal infections caused by dermatophytes parasitized on the stratum corneum of epidermis, follicles or nails. Dermatophytosis is a common disease, with about 20% of the world population having dermatophytosis, and one third of the total population in Europe is reported to be a patient with tinea pedis, and the prevalence rate is higher in the elderly than in the younger generation, dermatophytosis is a common and increasingly important disease that will become increasingly necessary at the present time to the aging population.

Dermatophytosis is classified into tinea pedis, tinea mannum, tinea capitis, tinea cruris, and tinea corporis depending on the site of development. Among them, Tinea pedis is divided into an interdigital type, hyperkeratotic type and vesicular type of infection according to the clinical symptoms. In the case of the hyperkeratotic type of tinea pedis, there are almost no symptoms of itching, but the treatment is not easy due to the difficulty of penetration of the drug due to chronic and thickened keratin. Such dermatophytosis is treated by oral or topical administration of an antifungal agent. The drugs administered orally include itraconazole, fluconazole, terbinafine, and the like. Some patients have hepatotoxicity such as liver dysfunction, when they are administered orally, thereby making it inconvenient to check liver function during drug administration.

Products such as clotrimazole, terbinafine, butenapine, luliconazole, and ketoconazole are used as local treatments, and because they are administered locally, they are not hepatotoxic because they do not go through liver metabolism. However, it is difficult to transdermally transfer the drug, in the case of severe fungal infections, and the chronic keratinized skin.

It is an important task for a person skilled in the art of developing a topical pharmaceutical product with low side effect of systemic exposure such as hepatotoxicity by effectively delivering the drug effectively to the skin tissue, as in oral administration. In addition, it is an important task for a person skilled in the art to develop a safe and effective drug to treat skin fungus, as oral administration of the drug, by delivering the drug to the skin, even when the drug is difficult to permeate due to the keratinization skin.

Therefore, various attempts have been made to maximize the delivery of antifungal drugs locally and reduce the administration frequency and treat diseases more effectively.

Korean Patent Publication No. 1996-0009415 discloses a pharmaceutical composition for the treatment of athlete's foot which contains croconazole as an antifungal substance and forms a coating film containing an ethyl acrylate-methyl methacrylate copolymer to maintain its drug efficacy for a long time. Korean Patent No. 10-1333892 discloses an antifungal drug comprising a terbinafine or a salt thereof, a film forming agent including a mixture of hydroxyalkyl cellulose and an acrylate copolymer, and a solvent, and having a fungal healing rate of 50% or higher in a single application.

Korean Patent No. 10-0979347 discloses a rapid-acting sustained-release antifungal composition comprising terbinafine or a salt thereof and containing trimethylsiloxysilicate to form a coating upon application of the skin. The invention increased the permeability by more than 10 times in the initial (within 12 hours) and more than 3 to 4 times in the latter period (within 72 hours) in Franz Diffusion cell equipped with nude mouse skin compared to Lamisil Once (Norvatis AG).

Korean Patent Laid-Open Publication No. 2012-0056314 discloses a topical antifungal composition containing terbinafine or a salt thereof and comprising a film forming agent, a solvent and a skin retention improving agent, wherein the skin retention improving agent is at least one member selected from the group consisting of urea, 1-dodecyl urea and 1,3-dodecyl urea. The document contains urea or 1-dodecyl urea and is characterized by significantly increasing the initial skin permeability compared to Lamisil Once (Norvatis AG) or increasing the amount of skin deposit by about 3 to 6 times. However, permeation enhancers such as urea have problems that cause side effects such as irritation symptoms such as pain and heat sensation, hypersensitivity, skin eczema and burning sensation.

As described in the above-mentioned patent documents, there have been many attempts to increase the drug efficacy by increasing the skin adhesion time of the antifungal agent, to represent the equivalent efficacy even when decreasing the administration frequency, or to increase the drug efficacy by increasing the drug penetrating rate. However, there is a need to develop an antifungal transdermal absorption preparation that maximizes skin permeability of antifungal agents, increases skin retention, reduces the administration frequency, and facilitates water washing. In addition, it is necessary to develop antifungal transdermal absorption agent, there has been no attempt to increase the skin permeation in the skin. Therefore, development of a pharmaceutical composition having the above characteristics is required.

### [Detailed Description of the Invention]

### [Technical Problem]

An embodiment of the present invention is to provide an antifungal transdermal formulation which is superior in drug delivery in hyper-keratinized skin having a difficulty absorbing drug, such as tinea or chronic angular athlete's foot, and increases the skin permeability of active substance in normal skin without permeation enhancer such as urea, and a method for producing the same.

### [Technical solution]

In order to achieve the object, the present invention provides an antifungal transdermal pharmaceutical composition comprising an antifungal agent, a hydrophilic film-forming agent, and a hydrophilic solvent, and a method of preparing the same.

The antifungal transdermal formulation of the present invention contains less than 1 wt% of hydrophobic additive such as a hydrophobic excipient, hydrophobic emulsifier, and the like, except for the antifungal agent, based on 100% by weight of the total pharmaceutical composition, or preferable does not contain of hydrophobic additive such as a hydrophobic excipient, hydrophobic emulsifier, and the like, except for the antifungal agent, without containing a permeation enhancer such as urea which has been used to increase the skin permeability and/or skin retention of the active material in conventional transdermal formulation containing antifungal agents.

The antifungal transdermal pharmaceutical composition of present invention has an excellent effect on hyper-keratinized skin such as intractable psoriasis and/or chronic keratinized athlete's foot which cannot be permeated deeply by drug, by the deep permeation of antifungal agent into skin at a high concentration. In addition, the composition can be absorbed by skin at a high concentration, thereby reducing the administration frequency. The composition can deliver drug in a short time by forming hydrophilic film at the lesion region, and can be easily washed well with water, thereby reducing the use inconvenience caused by the difficult washing.

In accordance with the present invention, it is to provide a method of increasing skin permeability of active ingredient, in order to increase the skin retention of drug in normal skin and the skin permeation of drug in hyper-keratinized skin such as intractable psoriasis and/or chronic keratinized athlete's foot which are not be permeated deeply by drug, including administering to a subject in need the antifungal transdermal composition containing an antifungal agent, a hydrophilic film-forming agent, and a hydrophilic solvent.

Hereinafter, the present invention will be described in more detail.

The antifungal pharmaceutical composition of the present invention contains an antifungal agent, a hydrophilic film-forming agent, a hydrophilic solvent, and a hydrophobic additive which is not an antifungal agent.

The composition according to the present invention may comprise at least one antifungal agent which may be hydrophobic material. The antifungal agent itself may be a free acid or free base, or salts thereof.

For example, the antifungal agent may be a 1-hydroxy-2-pyridone compound and a salt thereof, and a polyene derivative, an allylamine derivative, a triazole derivative, and their salts. Specifically, 1-hydroxy-2-pyridone compounds and salts thereof include, for examples ciclopirox, ciclopirox olamine, rilopirox, etc. The imidazole derivatives include, for examples clotrimazole, econazole, isoconazole, ketoconazole, myconazole, thioconazole, bifonazole, fenticonazole, oxiconazole, butoconazole, sertaconazole, sulconazole, luliconazole, omoconazole, and the like. The polyene derivatives include, for examples nystatin, natamycin, amphotericin and the like. Examples of the allylamine derivatives include naftifine, terbinafine, butenapine, amorolfine and the like. The triazole derivatives and salts thereof include, for examples albaconazole, efinaconazole, fluconazole, itraconazole, isavuconazole, labuconazole, posaconazole, voriconazole and terconazole. In addition, the antifungal agent may include at least one selected from the group consisting of avafungin, caspofungin, anidulafungin, micafungin, benzoic acid, haloprozine, ndecenoic acid, griseofulvin, tolnaftate and flucytosine.

A preferred example of the antifungal agent according to the present invention may include at least one selected from the group consisting of terbinafine, butenafine, ketoconazole, clotrimazole, cyclopirox, and pharmaceutically acceptable salts thereof.

The composition according to the present invention may contain 0.01 to 20 parts by weight, for example, 0.1 to 10 parts by weight or 0.2 to 5 parts by weight of an antifungal agent based on 100 parts by weight of the total composition.

In addition to antifungal agents, the composition according to the present invention may contain additional active substances for treating symptoms caused by the fungal infection. For example, the local anesthetics such as lidocaine, benzocaine, and dibucaine that alleviate pain by anesthetizing the lesion site, an itch inhibitor such as crotamiton, an antihistamine agent such as diphenhydramine that reduces the inflammatory response by reducing histamine, steroid or steroid-like substances such as enoxolone, glycyrrhizic acid, licorice extract, hydrocortisone and betamethasone that inhibit inflammation strongly, active substances such as menthol, campa, eucalyptol and the like that are easily volatilized at room temperature to remove itching and reducing itching and inflammation. For example, the pharmaceutical composition may further comprise one or more active substances selected from the group consisting of lidocaine, crotamiton, dipotassium glycyrrhizinate, enoxolone, diphenhydramine, diphenhydramine hydrochloride, thymol, and L-menthol.

The antifungal pharmaceutical composition for present invention includes a hydrophilic film-forming agent. The composition can be absorbed deeply at a high concentration in the skin, thereby reducing the administration frequency, form a hydrophilic film at the lesion site to deliver the drug in a short time, and is easily washed with water to reduce the use inconvenience caused by difficult washing.

The composition according to the present invention comprises at least one hydrophilic film-forming agent, which can form a film on the skin to increase drug absorption into the skin which is similar drug delivery to that of Occlusive Dressing Technique. The composition prevents the moisture from evaporating from the skin to loosen the connective tissue, thereby maximizing the transfer of the active substance into the skin. In addition, the formulation containing the drug can minimize the loss of the drug, since it does not get in with the contact object. The hydrophilic film-forming agent of the present invention may be a hydrophilic polymer. The hydrophilic polymer includes a water-soluble polymer that can be dissolved in water due to the functional group with polarity or charge in the unit molecule.

The hydrophilic film-forming agents being applicable to the present invention include at least one selected from the group consisting of hydrophilic or water-soluble cellulose derivatives, chitosan and chitosan derivatives, poloxamer, copolymers of polyoxypropylene and polyoxyethylene, copolymers of vinylcaprolactam/ vinylpyrrolidone/dimethylaminoethyl methacrylate, xanthan gum, acacia gum and locust bean gum. The hydrophilic cellulose derivatives include hydroxypropylmethylcellulose, hydroxypropylcellulose, and hydroxyethylcellulose. When the hydrophilic film-forming agent is chitosan, the pharmaceutical composition may further comprise an acid such as lactic acid being used for dissolving chitosan.

Preferably, the present invention may include at least one selected from the group consisting of chitosan, poloxamer, hydroxypropylmethylcellulose, hydroxypropylcellulose, and hydroxyethylcellulose, as the hydrophilic film-forming agent.

The composition according to the present invention may contain 0.5 to 20 parts by weight, for example, 0.5 to 18, or 0.5 to 10 parts by weight of a hydrophilic film-forming agent, based on 100 parts by weight of the total composition.

In the case of conventional transdermal formulation of antifungal agent, a water-insoluble polymer or a hydrophobic polymer has been largely used as a film forming agent for preventing the film formed on the skin from being washed and for delivering the active substance continuously. The present inventors have found that the hydrophobic additives including the water-insoluble polymer cause the drug to stay in the film rather than transfer the hydrophobic antifungal drug to the skin, thereby significantly reducing skin absorption, and have completed the present invention by not using the hydrophobic additives having washing difficulty or adding the hydrophobic additives in an amount of less than 1 wt%, because the drug is delivered initially in several hours in actual skin but not in vitro test condition.

A hydrophobic substance can be dissolved in a nonpolar solvent rather than a polar solvent and be hardly dissolved in a hydrophilic solvent, because of a nonpolar property. In the present invention, the hydrophobic additive may be a pharmaceutically acceptable additive having a hydrophobic property and does not include a hydrophobic active material or an active ingredient. For example, the hydrophobic additive may be at least one selected from the group consisting of a hydrophobic carrier, a hydrophobic emulsifier, a hydrophobic auxiliary emulsifier, hydrophobic excipients, hydrophobic film-forming agent, hydrophobic binders, and the like.

Examples of the hydrophobic additives are at least one selected from the group consisting of yellow bee wax, white bee wax, oils such as Vaseline (petrolatum jelly), mineral oil, paraffin, lanolin, castor oil, olive oil, canola oil, corn germ oil and sunflower oil; fatty alcohols such as lauryl alcohol, myristyl alcohol, cetyl alcohol, stearyl alcohol, cetostearyl alcohol, behenyl alcohol and the like; fatty acids such as capric acid, lauric acid, myristic acid, palmitic acid, stearic acid, arachidic acid, behenic acid, alpha linolenic acid, oleic acid, gamma linolenic acid and eicopentaenoic acid and the like; fatty acid esters such as isopropyl myristate, propionic acid isostearate, isopropyl palmitate, glyceryl monooleate, glyceryl monostearate, glyceryl palmitostearate, glyceryl behenate, cetyl palmitate, octyldodecanol, medium chain fatty acid triglyceride, long chain fatty acid triglyceride (hard fat) and the like; siloxane compounds such as dimethicone, methyltrimethicone, cyclomethicone, dimethiconol, hydrogendimethicone, and methicone; alkyl olefinate, alkyl olefinate copolymer, amide/olefinic acid, amide/olefinic acid copolymer, polyvinylacetate, ethylcellulose, alkylacrylate copolymer as acrylate polymers or acrylate copolymers, octyl acrylamide, acrylamide/acrylate copolymer, octylpropeneamide acrylate copolymer, ethyl acrylate/methacrylate copolymer, aminoalkylmethacrylate copolymer, aammonium methacrylate copolymer, PVM/MA copolymer, PVP/VA copolymer, PVA, methylvinyl ether and ethyl maleic acid maleate, isopropylmaleate and butylmaleate copolymer, Shellac, hydroxypropylcellulose phthalate, hydroxypropylcellulose acetate succinate, acrylate/dimethicone copolymers, trimethylsiloxysilicate, polyvinyl acetal diethylaminoacetate, polysorbate 60 and sorbitan monosorbate.

Preferably, the hydrophobic additive is selected from the group consisting of paraffin, cetostearylalcohol, isopropylmyristate, cetylpalmitate, dimethicone, polysorbate 60, sorbitan monosorbate, acrylamide/acrylate copolymer, aminoalkylmethacrylate copolymer, PVM/MA copolymer, trimethylsiloxysilicate and polyvinyl acetal diethylaminoacetate.

Since the hydrophobic additive dissolves the antifungal agent in a hydrophobic substance to inhibit migration of the antifungal agent to the skin, the amount of the hydrophobic additive should be limited, and preferable may be less than 1 wt%.

In the composition according to the present invention, the hydrophobic additive does not include the antifungal substance, but can be contained at an amount of less than 1% by weight, such 0.5% by weight or less, 0.1% by weight or less, 0.05% by weight or less and 0.001% by weight or less, based on 100% by weight of the total composition. Preferably, the composition according to the present invention does not include the hydrophobic additive.

The composition according to the present invention may need a solvent for dissolving an antifungal agent and a film-forming agent. Examples of the solvents include at least one selected from the group consisting of water, a lower alcohol having 1 to 6 carbon atoms, and acetone. The lower alcohol may be ethanol, 1-propanol, 2-propanol, 1-butanol, 2-butanol, 2-methyl-1-propanol, 1-pentanol and 3-methyl-1-butanol. Preferably, the solvent may be a mixture of alcohol and water.

The solvent according to the present invention may be 20 to 99 parts by weight, for example, 60 to 99 parts by weight or 60 to 98 parts by weight, which is an amount sufficient to dissolve the active material and the film-forming agent. In addition to the components described above, the pharmaceutical composition of the present invention can contain additional and conventionally-used plasticizers, thickeners, surfactants, antioxidants, auxiliary solvents, preservatives, flavoring agents and the like, which are used for improving convenient administration, stability of formulation and the drug permeation. The examples are well illustrated in the Handbook of Pharmaceutical Excipients 7th edition (Raymond C. Rowe).

The drug permeation amount of the transdermal formulation according to the present invention can be measured for normal skin and hyper-keratinized skin, and the drug amount per skin gram (g) to be tested is referred to the skin retention amount of drug (mcg/g). In addition, the drug amount permeated unit area (cm²) of the skin can be indicated as cumulative skin permeation amount (mcg/cm²) of the drug. The skin retention amount or the cumulative skin permeation amount of the drug can be obtained by various methods known to those skilled in the art.

For example, the drug permeation amount of a transdermal formulation can be measured using normal skin tissue, for example pig skin. Specifically, pig skin is placed in a Franz-diffusion cell with a diameter of 9 mm, 300 mg of 1% composition of the present invention is filled to a donor, and 5 ml of PBS (pH 5.8) is filled to a receptor. Then, while stirring the cell, the skin is cut at a predetermined time and pulverized with a Cell-lyser. The remaining active substance in the skin was quantitated by HPLC to quantify the antifungal agent delivered to the skin. The skin retention amount of drug may be 600 (mcg/g) or more, for example 1000 (mcg/g) or more or 1500 (mcg/g) or more. The skin retention amount of drug is 600 (mcg/g) to 3000 (mcg / g), or 1500 (mcg/g) to 3000 (mcg/g).

The antifungal transdermal formulation according to the present invention can penetrates an antifungal agent deeply into the skin at a high concentration and thus be applied to a chronic aphrodisiac type athlete's foot or a tinea which cannot be easily permeated by drug.

As another example, the drug permeation amount of a transdermal formulation can be measured using the keratinized skin tissue which is induced from mouse skin. For example, the skin sample is obtained from NC/Nga mouse skin with the induced keratinization, and added to Franz-diffusion cell with a diameter of 9 mm. 300mg of 1% composition of the present invention is added to donor and 5 ml of 50% EtOH is filled into receptor. The sample is taken from the receptor at a determined time with agitating, and is tested for measuring the amount of antifungal agent penetrated into the skin sample with HPLC. The measured cumulative permeation amount of drug into the skin may be represented as the cumulative permeation amount of drug into the skin of 600 (mcg/cm²) or higher, or 1000(mcg/cm²) or higher, which is measured at 24 hours after being applied by the transdermal composition of the present invention.

### [Advantageous effect]

The transdermal absorption formulation containing the antifungal agent according to the present invention can penetrates an antifungal agent deeply into the skin at a high concentration, thereby treating a chronic aphrodisiac type athlete's foot or a tinea which does not easily delivered by the drug. It is possible to reduce the inconvenience of daily administration by retention of the drug at a high concentration in the skin. The transdermal absorption formulation forms a hydrophilic film on the lesion site, to deliver the drug in a short time and to be easily washed with water, so as to remove the difficulty of washing the film.

### [Brief Description of Drawings]

FIG. 1 is a graph showing the result of measuring the skin retention amount of a transdermal formulation obtained in the examples and comparative examples, by using pig skin.

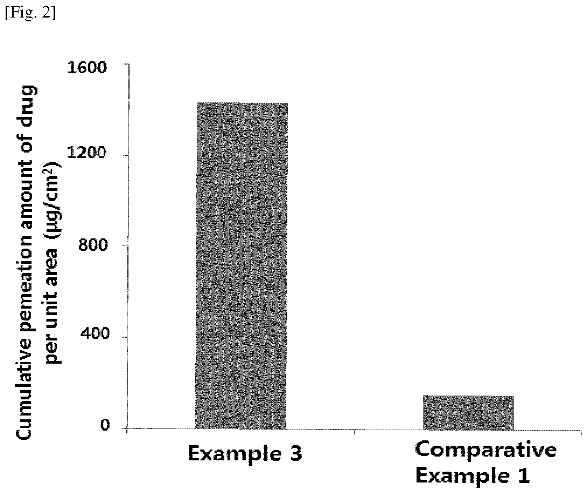

FIG. 2 is the comparison of the skin permeability in hyper-keratinized skin for the transdermal formulation according to the example and comparative example.

### [Mode of the Invention]

Hereinafter, the present invention will be described in detail with reference to examples. However, the following examples are illustrative of the present invention, and the contents of the present invention are not limited by the following examples

### Example 1-4: Preparation of antifungal transdermal formulation (1)

According to the components and the mixing ratio of Table 1, the antifungal formulations were prepared. Specifically, EXAMPLE 1 contained terbinafine as an antifungal agent and chitosan as a hydrophilic film-forming agent. Example 2 contained terbinafine hydrochloride as an antifungal agent and chitosan as a hydrophilic film-forming agent. EXAMPLE 3 contained terbinafine as an antifungal agent, and a mixture of chitosan and hydroxypropylcellulose as a hydrophilic film-forming agent. EXAMPLE 4 contained butenafine hydrochloride as an antifungal agent and chitosan and hydroxypropylcellulose as hydrophilic film-forming agents. Propylene glycol was used as a plasticizer or an auxiliary solubilizer, and benzyl alcohol was used as a preservative. The hydrophilic solvent contained 95% ethanol aqueous solution and distilled water.

Specifically, ethanol and lactic acid were added to dissolve the antifungal agent, and propylene glycol and benzyl alcohol were dissolved therein. Then, chitosan and hydroxypropylcellulose were added thereto, and purified water was added thereto so as to adjust the total weight of the percutaneous preparation to 100 wt%. Then, the resulting solution was agitated with homomixer for 1 hour, to produce a homogeneous composition.

**[Table 1]**

| ingredient | EXAMPLE 1 | EXAMPLE2 | EXAMPLE3 | EXAMPLE 4 |
|---|---|---|---|---|
| Terbinafine | 1 | - | 1 | - |
| Terbinafine hydrochloride | - | 1.125 | - | - |
| Butenafine hydrochloride | - | - | - | 1 |
| Chitosan | 2 | 2 | 1 | 1 |
| Hydroxypropylcellulose | - | - | 1 | 1 |
| Lactic acid | 4.8 | 3.4 | 3.5 | 4 |
| Propylene glycol | 1 | 1 | - | - |
| Benzyl alcohol | 0.5 | 0.5 | 0.5 | 0.5 |
| 95% ethanol | 38.5 | 37.5 | 41.5 | 41 |
| Distilled water | Appropriate amount | Appropriate amount | Appropriate amount | Appropriate amount |
| Total (weight %) | 100 | 100 | 100 | 100 |

### Examples 5-12: Preparation of antifungal transdermal formulation

Antifungal transdermal formulations were prepared using the ingredients and mixing ratios shown in Table 2.

In Examples 5 to 8, terbinafine hydrochloride was used as an antifungal agent. As the hydrophilic film-forming agent, hydroxypropylcellulose was used in Example 5, hydroxyethylcellulose was used in Example 6, hydroxypropylmethylcellulose was used in Example 7, and poloxamer was used in Example 8. Specifically, the antifungal agent was dissolved in ethanol, and added by propylene glycol and benzyl alcohol. Then, the hydrophilic film-forming agent and water were added to adjust 100 wt% and stirred for 1 hour with a homomixer to prepare a homogeneous composition.

In Examples 9 to 12, ketoconazole, clotrimazole, luliconazole, or cyclopirox olamine were used as antifungal agents, and hydroxypropylcellulose and hydroxyethylcellulose were mixed as a hydrophilic film-forming agent. Specifically, the compositions of EXAMPLES 9 and 10 were prepared in the same manner as EXAMPLE 5, except that the antifungal agent was dissolved in ethanol and benzyl alcohol as solvent.

The compositions of EXAMPLEs 11 and 12 were prepared in the same manner as EXAMPLE 5, except that the antifungal agent was dissolved in isopropyl alcohol and benzyl alcohol as solvent. The composition of EXAMPLE 8 is prepared in the same manner as EXAMPLE 5, except that the antifungal agent was dissolved in ethanlol, isopropyl alcohol and lactic acid.

**[Table 2]**

| Ingredient | EXAMPLE 5 | EXAMPLE 6 | EXAMPLE 7 | EXAMPLE 8 | EXAMPLE 9 | EXAMPLE 10 | EXAMPLE 11 | EXAMPLE 12 |
|---|---|---|---|---|---|---|---|---|
| Terbinafine hydrochloride | 1.125 | 1.125 | 1.125 | 1.125 | | - | - | - |
| Ketoconazole | - | - | - | - | 2 | - | - | - |
| Clotrimazole | - | - | - | - | - | 1 | - | - |
| Luliconazole | - | - | - | - | - | | 1 | - |
| Ciclopiroxolamine | - | - | - | - | - | | | 1 |
| Hydroxypropyl cellulose | 2.5 | - | - | - | 1 | 1 | 1 | 1 |
| Hydroxyethyl cellulose | - | 2.5 | - | - | 1 | 1 | 1 | 1 |
| Hydroxypropylmethylcellulose | - | - | 2.5 | - | - | - | - | - |
| Poloxamer F127 | - | - | - | 5 | - | - | - | - |
| Propylene glycol | 1 | 1 | 1 | 1 | - | - | - | - |
| Benzyl alcohol | 0.5 | 0.5 | 0.5 | - | 0.5 | 0.5 | - | - |
| 95% ethanol | 41 | 25 | 36.5 | 24.5 | 75 | 85 | - | - |
| Isopropylalcohol | - | - | - | - | - | - | 90 | 90 |
| Distilled water | Appropriate amount | Appropriate amount | Appropriate amount | Appropriate amount | Appropriate amount | Appropriate amount | Appropriate amount | Appropriate amount |
| Total(100 wt%) | 100 | 100 | 100 | 100 | 100 | 100 | 100 | 100 |

### EXAMPLE 13-17: Formulations containing an antifungal agent and an active substance alleviating tinnitus symptoms

In according with the ingredients and their amounts in Table 3, the compositions containing an antifungal agent and an active substance for alleviating tinea were prepared. In Examples 13 to 17, terbinafine hydrochloride was used as an antifungal agent and lidocaine, crotamiton, dipotassium glycyrrhizinate, enoxolone, diphenhydramine, diphenhydramine hydrochloride, thymol or L-menthol were used in combination as an active substance for alleviating tinea .

Specifically, in Example 13, an antifungal agent and a tinea-related active substance were dissolved in ethanol and lactic acid, and mixed with the addition of benzyl alcohol and 95% ethanol aqueous solution. Then, the hydrophilic film-forming agent and water were added to adjust the total weight of composition to 100% by weight, and the mixture was stirred with a homomixer for 1 hour to prepare a homogeneous composition.

The compositions of EXAMPLEs 14 to 16 were prepared according to the same method of Example 13, except that an antifungal agent and a tinea-related active substance were dissolved in ethanol and benzyl alcohol. The composition of EXAMPLE 17 was prepared according to the same method of Example 13, except that an antifungal agent and a tinea-related active substance were dissolved in benzyl alcohol.

**[Table 3]**

| Ingredient | EXAMPLE 13 | EXAMPLE 14 | EXAMPLE 15 | EXAMPLE 16 | EXAMP LE17 |
|---|---|---|---|---|---|
| Terbinafine hydrochloride | 1 | 1 | 1 | 1 | 1 |
| lidocaine | 2 | 2 | 2 | 2 | 2 |
| crotamitone | - | - | - | 5 | - |
| Dipotassium glycyrrhizinate | 0.5 | - | - | 0.5 | - |
| Enoxolone | - | 0.1 | 0.5 | - | 0.5 |
| diphenhydramine | - | 1 | - | - | - |
| diphenhydramine hydrochloride | - | - | - | - | 0.5 |
| Thymol | | - | 0.3 | | 0.3 |
| L-menthol | 1 | - | 2 | 2 | - |
| chitosan | 1 | - | - | - | - |
| Hydroxyethylcellulose | - | - | 2 | - | - |
| Hydroxypropylcellulose | 1 | 2 | - | 2 | 2 |
| Poloxamer F127 | - | 0.5 | 0.5 | - | - |
| Lactic acid | 1 | - | - | - | - |
| Benzyl alcohol | 0.5 | 0.5 | 0.5 | - | - |
| 95% ethanol | 40 | 55 | 45 | 60 | - |
| Isopropyl alcohol | - | - | - | - | 60 |
| Distilled water | Appropriate amount | Appropriate amount | Appropriate amount | Appropriate amount | Appropri ate amount |
| sum (wt%) | 100 | 100 | 100 | 100 | 100 |

### COMPARATIVE EXAMPLES 1-3: Formulations containing a hydrophilic film-forming agent and a hydrophobic additive

According to the components and the composition of Table 4, the antifungal transdermal formulations containing both a hydrophilic film-forming agent and a hydrophobic additive were prepared. The formulations were prepared by the methods exemplified in conventional pharmaceutical manufacturing methods, e.g., Handbook of Pharmaceutical Manufacturing Formulations (Sarfaraz K. Niazi, CRC Press).

**[Table 4]**

| Ingredient | COMPARATIVE EXAMPLE 1 | COMPARATIVE EXAMPLE 2 | COMPARATIVE EXAMPLE 3 |
|---|---|---|---|
| Terbinafine hydrochloride | 1.125 | 1.125 | 1.125 |
| Caprylic / capric triglyceride | 3 | - | - |
| Isopropyl myristate | - | 1.375 | - |
| Dimethicone | - | 4.8 | - |
| Trimethylsiloxysilicate | - | 7.2 | - |
| PVM/MA copolymer (Gantrez AN) | - | - | 1 |
| Octyl acrylamide/acrylate copolymer (Dermacryl 79) | 5 | - | - |
| L-menthol | - | 3 | - |
| Chitosan | - | - | 2 |
| Lactic acid | - | - | 2 |
| Hydroxypropylcellulose | 1 | 2.5 | |
| 95% ethanol | 89 | 80 | 50 |
| Distilled water | Appropriate amount | Appropriate amount | Appropriate amount |
| Total(wt%) | 100 | 100 | 100 |

### COMPARATIVE EXAMPLES 4-10: Formulation containing a hydrophobic additive

Antifungal transdermal formulations containing an emulsifying agent, an emulsifying aid or a film-forming agent as hydrophobic excipients were prepared, according to the components and mixing ratios shown in Table 5. The formulations were prepared by the methods exemplified in conventional pharmaceutical manufacturing methods, e.g., Handbook of Pharmaceutical Manufacturing Formulations (Sarfaraz K. Niazi, CRC Press).

Examples of the hydrophobic polymer include octylacrylamide/acrylate, PVM/MA copolymer, polyvinylacetal diethylaminoacetate, and aminoalkyl methacrylate copolymer. Examples of the hydrophobic emulsifying agent or emulsifying aid were cetostearylalcohol, cetylpalmitate, isopropylmyristate, polysorbate 60, and sorbitan monosorbate were used.

Specifically, 1wt% of octylacrylamide/acrylate copolymer (Dermacryl 79) in Comparative example 4, 1wt% of PVM/MA copolymer (gantrez AN) in Comparative example 5, 1wt% of polyvinylacetal diethylaminoacetate in Comparative example 6, 1 wt% of aminoalkyl methacrylate copolymer (eudragit E) in Comparative example 7 were used.

Comparative example 8 used 10 wt% of isopropyl myristate and 5 wt% of polysorbate 60. Comparative example 9 contained 8 wt% of cetostearyl alcohol, 2 wt% of cetyl palmitate, 4 wt% of polysorbate 60 and 1 wt% of sorbitan monosorbate. Comparative example 10 contained 13 wt% of paraffin, 10 wt% of cetostearyl alcohol, 3 wt% of polysorbate 60, and 2 wt% of sorbitan monosorbate.

**[Table 5]**

| Component | COMPARATIVE EXAMPLE 4 | COMPARATIVE EXAMPLE 5 | COMPARATIVE EXAMPLE 6 | COMPARATIVE EXAMPLE 7 | COMPARATIVE EXAMPLE 8 | COMPARATIVE EXAMPLE 9 | COMPARATIVE EXAMPLE 10 |
|---|---|---|---|---|---|---|---|
| Terbinafine hydrochloride | 1.125 | 1.125 | 1.125 | 1.125 | - | 1.125 | 1.125 |
| Terbinafine | - | - | - | - | 1 | - | - |
| Hydrophobic polymer | 1 | 1 | 1 | 1 | - | - | - |
| paraffin | - | - | - | - | - | | 13 |
| Cetostearyl alcohol | - | - | - | - | - | 8 | 10 |
| Cetyl palmitate | - | - | - | - | | 2 | |
| Isopropyl myristate | - | - | - | - | 10 | - | - |
| Polysorbate 60 | - | - | - | - | 5 | 4 | 3 |
| Sorbitan monosorbate | - | - | - | - | 1 | 1 | 2 |
| Carbomer | - | - | - | - | 1 | - | - |
| Sodium hydroxide | - | - | - | - | 0.2 | 0.1 | - |
| Benzyl alcohol | - | - | - | - | 5 | - | 5 |
| 95% ethanol | 94 | 94 | 94 | 94 | 15 | 10 | - |
| Distilled water | Appropriate amount | Appropriate amount | Appropriate amount | Appropriate amount | Appropriate amount | Appropriate amount | Appropriate amount |
| Total (wt%) | 100 | 100 | 100 | 100 | 100 | 100 | 100 |

### Experimental Example 1: Film Formation and Washing Experiment

When the transdermal formulations of Examples 1 to 17 were applied to the skin, they were dried after 3 to 5 minutes to form a thin film. The resulting film was easily washed in 5 to 10 minutes by washing with water at a rate of 100 ml/min using a peristaltic pump.

### Experimental Example 2: Comparison of drug concentration in skin

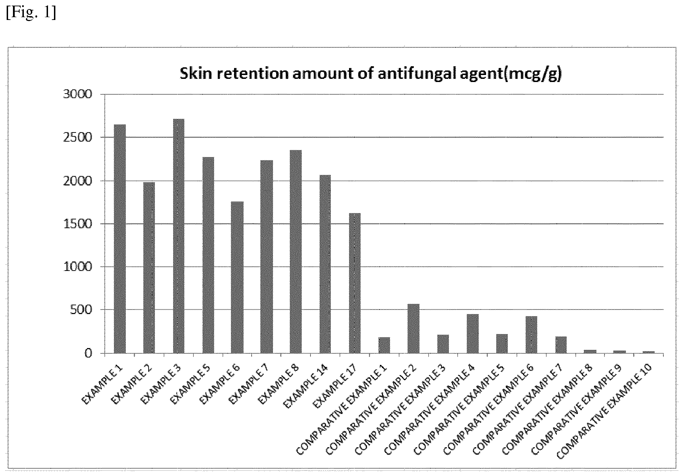

The pig skin was cut to a constant thickness and mounted on a Franz diffusion cell. The receptor portion was filled with PBS (pH 5.8) and the donor portion was treated with the pharmaceutical composition of Examples 1 to 7, Example 14, Example 17 and Comparative Examples at an amount of 1 to 9 300 micrograms. After 24 hours, the skin was detached from the apparatus and washed. The certain area of skin was cut and weighed. Then, the skin tissue was dissolved using 2N sodium hydroxide solution and a cell lyser, and the active ingredient was extracted with ethyl acetate. Ethyl acetate was evaporated from the obtained extract solution, and then dissolved using a mobile phase for HPLC analysis. Then, the concentration of the active ingredient was determined with HPLC analysis. The results are shown in Table 6 and FIG. 1, which are the results of measuring skin retention amount of the antifungal agent as an active ingredient.

**[Table 6]**

| Examples | Skin retention amount of antifungal agent(mcg/g) | deviation |
|---|---|---|
| EXAMPLE 1 | 2652 | 146 |
| EXAMPLE 2 | 1980 | 280 |
| EXAMPLE 3 | 2720 | 210 |
| EXAMPLE 5 | 2268 | 153 |
| EXAMPLE 6 | 1752 | 198 |
| EXAMPLE 7 | 2234 | 573 |
| EXAMPLE 8 | 2356 | 367 |
| EXAMPLE 14 | 2068 | 220 |
| EXAMPLE 17 | 1625 | 210 |
| COMPARATIVE EXAMPLE 1 | 178 | 25 |
| COMPARATIVE EXAMPLE 2 | 570 | 114 |
| COMPARATIVE EXAMPLE 3 | 211 | 15 |
| COMPARATIVE EXAMPLE 4 | 456 | 140 |
| COMPARATIVE EXAMPLE 5 | 221 | 34 |
| COMPARATIVE EXAMPLE 6 | 422 | 44 |
| COMPARATIVE EXAMPLE 7 | 187 | 24 |
| COMPARATIVE EXAMPLE 8 | 35 | 5 |
| COMPARATIVE EXAMPLE 9 | 31 | 2 |
| COMPARATIVE EXAMPLE 10 | 15 | 2 |

As shown in Table 6 and FIG. 1, it was confirmed that the formulations of Examples significantly increased the concentration of the active substance retained in the skin, compared to the Comparative examples containing the hydrophobic additive.

### Experimental Example 3: Drug skin permeability to hyper-keratinized skin

NC/Nga mice were treated with 2,4-dinitrochlorobenzene for 2 weeks to induce hyper-keratinized skin, and sacrificed to obtain the mouse hyper-keratinized skin. The obtained hyper-keratinized was cut into a predetermined area and attached to a Franz diffusion cell. 50% aqueous ethanol solution was applied to the receptor and 300 micrograms of formulations in Example 3 and Comparative example 1 were added to the donor. At 24 hours, the amount of active ingredient in the receptor was analyzed using HPLC. As a result, the composition of Example 3 had a cumulative drug permeation amount of 1432 (mcg/cm²) and the composition of Comparative example 1 had a cumulative drug permeation amount of 155 (mcg/cm²).

The composition according to the present invention showed that the skin permeability of the active ingredient was remarkably increased even in the hyper-keratinized skin.

## Claims

1. An antifungal transdermal pharmaceutical composition comprising an antifungal agent, a hydrophilic film-forming agent and a hydrophilic solvent, wherein the pharmaceutical composition comprises less than 1% by weight of a hydrophobic additive based on the total weight of the composition.

2. The antifungal transdermal pharmaceutical composition of claim 1, wherein skin retention amount of the antifungal agent of the composition 600 (mcg/g) or higher.

3. The antifungal transdermal pharmaceutical composition of claim 2, wherein skin retention amount of the antifungal agent of the composition 1,500 (mcg/g) or higher.

4. The pharmaceutical composition of claim 1, wherein the composition is applied for hyperkeratinized skin.

5. The pharmaceutical composition of claim 4, wherein the composition is applied for tinea or chronic keratinized athlete's foot.

6. The pharmaceutical composition of claim 1, wherein the hydrophobic additive is at least one selected from the group consisting of a hydrophobic carrier, a hydrophobic emulsifier, a hydrophobic auxiliary emulsifying, a hydrophobic excipient, a hydrophobic film-forming agent and a hydrophobic binder.

7. The pharmaceutical composition of claim 1, wherein the hydrophilic film-forming agent is selected from the group consisting of hydroxyethyl cellulose, hydroxypropyl cellulose, hydroxypropylmethyl cellulose, chitosan, a copolymer of polyoxypropylene and polyoxyethylene, a copolymer of vinylcaprolactam/vinylpyrrolidone/ Dimethylaminoethyl methacrylate, xanthan gum, acacia gum, and locust bean gum.

8. The pharmaceutical composition of claim 1, wherein the antifungal agent is at least one selected from the group consisting of Terbinafine, butenapine, ketoconazole, clotrimazole, cyclopirox, and pharmaceutically acceptable salts thereof.

9. The pharmaceutical composition of claim 1, wherein the pharmaceutical composition comprises at least one second active substance selected from the group consisting of lidocaine, crotamiton, dipotassium glycyrrhizinate, enoxolone, diphenhydramine, diphenhydramine hydrochloride, thymol and L-mentol.

10. The pharmaceutical composition of claim 1, wherein the hydrophilic solvent is at least one selected from the group consisting of water and a lower alcohol having 1 to 6 carbon atoms.

11. The pharmaceutical composition o claim 10, wherein the alcohol is ethanol or isopropyl alcohol

12. The pharmaceutical composition of claim 1, wherein the pharmaceutical composition comprises, based on 100 parts by weight of the total amount of the pharmaceutical composition, 0.2 to 5 parts by weight of an antifungal agent, 0.5 to 10 parts by weight of a hydrophilic film-forming agent, and 60 to 98 parts by weight of a hydrophilic solvent.

13. The pharmaceutical composition of claim 1, wherein the composition is in the form of a liquid, a gel or an ointment.
